# FASCICULE DE BREVET EUROPEEN

(11) **EP 0 693 926 B1**
(45) Date de publication et mention de la délivrance du brevet: **15.01.1997**
(21) Numéro de dépôt: 95902174.2
(22) Date de dépôt: 25.11.1994
(51) Int. Cl.: A61K 31/165, A61K 31/60, A61K 31/615, A61K 9/00

(54) **NOUVELLE COMPOSITION PHARMACEUTIQUE DESTINEE A LA PREPARATION D'UNE POUDRE STABLE CONTENANT, A TITRE D'INGREDIENT ACTIF, UNE ASSOCIATION D'ACIDE ACETYLSALICYLIQUE ET DE METOCLOPRAMIDE**
Arzneizubereitung zum Herstellen eines stabilisierten Pulvers das eine Kombination aus Acetylsalicylsäure und Metoclopramid als Wirkstoff enthält
NOVEL PHARMACEUTICAL COMPOSITION FOR THE PREPARATION OF A STABLE POWDER CONTAINING AN ACTIVE PRINCIPLE COMPRISING AN ASSOCIATION OF ACETYLSALICYLIC ACID AND METOCLOPRAMIDE

(30) Priorité: 26.11.1993 FR 9314170
(43) Date de publication de la demande: 31.01.1996
(73) Titulaire: LABORATOIRES UPSA, 47000 Agen (FR)
(72) Inventeur: BRU, Nicole, F-75016 Paris (FR); CORDOLIANI, Jean-François, F-47390 Layrac (FR); POLY, Pierre-André, F-91200 Athis Mons (FR); DROUIN, Jehan-Yves, F-91370 Verrières-le-Buisson (FR)
(74) Mandataire: Hubert, Philippe
(86) Numéro de dépôt international: FR9401374
(87) Numéro de publication internationale: WO9514462

(56) Documents cités:
- EP-A- 0 011 489
- EP-A- 0 011 490
- DATABASE WPI Week 9212, Derwent Publications Ltd., London, GB; AN 92-088747 (12)
- CHEMICAL ABSTRACTS, vol. 101, no. 17, 22 Octobre 1984, Columbus, Ohio, US; abstract no. 143531p,

## Description

La présente invention a pour objet une nouvelle composition pharmaceutique contenant, à titre d'ingrédient actif, une association d'un sel ou complexe soluble dans l'eau de l'acide acétylsalicylique et du métoclopramide ou de l'un de ses sels pharmaceutiquement acceptables.

L'invention trouve notamment application pour la préparation d'un médicament destiné au traitement de la migraine, se présentant sous forme de poudres effervescentes ou non.

La migraine est une affection bénigne, affectant 5 à 25 % de la population adulte, qui se caractérise par des accès répétitifs de céphalées, unilatéraux et très souvent associés à des nausées et vomissements qui augmentent considérablement l'inconfort dû aux céphalées.

L'acide acétylsalicylique, anti-inflammatoire non-stéroïdien, entre dans l'éventail des thérapeutiques anti-migraineuses grâce à ses propriétés antalgiques.

Cependant, plusieurs études de pharmacocinétique ont permis de mettre en évidence, lors de la crise de migraine, un retard à l'absorption de l'acide acétylsalicylique, dû à une stase gastrique ainsi qu'une diminution de la quantité d'acide acétylsalicylique absorbée.

L'administration du métoclopramide ou 4-amino-5-chloro-2-méthoxy-N-(β-diéthylaminoéthyl)benzamide, en association avec l'acide acétylsalicylique a permis de remédier à ce problème.

On a en effet constaté que le métoclopramide, antagoniste dopaminergique présentant une activité anti-émétique, agit sur la stase gastrique et le ralentissement de l'évacuation gastrique, phénomènes responsables de l'altération de la vitesse d'absorption de l'acide acétylsalicylique, et caractérisant les crises de migraine.

Cette association de l'acide acétylsalycilique et du métoclopramide a fait l'objet de nombreuses études, et a conduit à la commercialisation, notamment au Royaume-Uni, d'un comprimé connu sous le nom de MIGRAVESS®.

Cependant, il n'existe pas, à l'heure actuelle, d'association d'acide acétylsalicylique et de métoclopramide sous forme de poudre.

Les présents inventeurs ont découvert que les formes pulvérulentes associant l'acide acétylsalicylique et le métoclopramide sont instables et conduisent notamment à la formation du N-acétylmétoclopramide.

Dans ces conditions, la présente invention a pour but de résoudre le problème technique consistant en la fourniture d'une nouvelle composition pharmaceutique destinée à la préparation d'une poudre stable contenant à titre d'ingrédient actif une association d'acide acétylsalicylique et de métoclopramide.

Les études entreprises ont montré qu'il était possible de réaliser une composition pharmaceutique répondant à cet objectif :
- d'une part, en utilisant l'acide acétylsalicylique sous la forme d'un sel ou complexe soluble, et
- d'autre part, en ajoutant à cette association au moins un polymère hydrophile, en une quantité suffisante pour stabiliser le métoclopramide.

Ainsi, selon un premier aspect, la présente invention a pour objet une composition pharmaceutique destinée à la préparation d'une poudre, caractérisée en ce qu'elle comprend à titre d'ingrédient actif, une quantité efficace d'un sel ou complexe soluble dans l'eau de l'acide acétylsalicylique en association avec du métoclopramide ou l'un de ses sels pharmaceutiquement acceptables ; et au moins un polymère hydrophile pharmaceutiquement acceptable, en une quantité suffisante pour stabiliser le métoclopramide.

Dans le cadre de la présente invention, divers sels ou complexes solubles dans l'eau de l'acide acétylsalicylique peuvent être utilisés, comme par exemple
- le carbasalate de calcium;
- l'acétylsalicylate de lysine;
- l'acétylsalicylate de sodium.

Cependant, les meilleurs résultats ont été obtenus avec le carbasalate de calcium.

Ce composé également connu sous le nom de carbasalate calcique est un complexe précurseur de l'acide acétylsalicylique comprenant deux molécules d'acide acétylsalicylique stabilisées par une molécule d'urée et un atome de calcium.

Ce composé a été parfaitement décrit dans l'état de la technique (MERCK INDEX 11th. Edition, p 250).

De même, dans le cadre de la présente invention, divers polymères hydrophiles pharmaceutiquement acceptables peuvent être utilisés comme par exemple, la polyvidone, des dérivés de cellulose (hydroxypropylcellulose, hydroxypropylmethylcellulose, hydroxyethylcellulose, carboxymethylcellulose) la gomme xanthane.

Les meilleurs résultats ont été obtenus en utilisant la polyvidone, ou polyvinylpyrrolidone, comme polymère hydrophile.

Le métoclopramide entrant dans la composition conforme à la présente invention, se présentera généralement sous forme libre, ou sous la forme de dichlorohydrate ou de monochlorohydrate.

Les meilleurs résultats ont été obtenus en utilisant le monochlorhydrate de métoclopramide.

Les poudres susceptibles d'être obtenues dans le cadre de la présente invention, peuvent être effervescentes ou non.

Une composition, selon l'invention, destinée à la préparation d'une poudre effervescente, comprendra un système effervescent pharmaceutiquement acceptable comprenant au moins un acide organique et au moins une substance susceptible de réagir avec cet acide organique pour produire, en présence d'une quantité suffisante d'eau,un dégagement de dioxyde de carbone.

Les acides susceptibles d'être utilisés à cet effet sont par exemple l'acide citrique, l'acide fumarique, l'acide adipique, l'acide tartrique ainsi que des mélanges de ces composés.

Un acide particulièrement préféré est l'acide citrique.

Parmi les substances susceptibles de réagir avec ces acides organiques, on citera en particulier les carbonates alcalins, comme notamment le bicarbonate de sodium, le bicarbonate de potassium, le carbonate de sodium, le carbonate de potassium ; et également la carboxylysine, le carbonate de calcium et les mélanges des composés précités.

Avantageusement, on utilisera le bicarbonate de sodium.

Les quantités relatives d'acide et de composé susceptible de réagir avec cet acide pourront être facilement déterminées par l'homme de métier selon l'effet effervescent recherché.

Dans un mode de réalisation préféré où le système effervescent est constitué d'un mélange de bicarbonate de sodium et d'acide citrique, les proportions en poids relatives de ces composés au sein du système effervescent seront comprises entre 20:80 et 80:20.

Selon une caractéristique particulière de l'invention, cette composition comprend en outre une quantité efficace d'au moins un déshydratant interne.

La présence d'un déshydratant interne au sein de cette composition permet de renforcer la stabilité de la poudre susceptible d'être préparée à partir de cette composition.

Le déshydratant interne a pour fonction de piéger les traçes d'eau natives ou susceptibles d'apparaître éventuellement dans la poudre lors de la préparation ou lors de la conservation du produit.

Avantageusement, on utilisera le citrate de magnésium ou le carbonate disodique à titre de déshydratant interne.

Selon une autre caractéristique particulière de l'invention, cette composition comprend en outre un diluant pharmaceutiquement acceptable.

Ce diluant joue également un rôle favorable dans la stabilisation des poudres susceptibles d'être préparées à partir de ces compositions.

Tout diluant pharmaceutiquement acceptable peut être utilisé dans la cadre de la présente invention, comme en particulier le sucrose, le dextrose, le mannitol, le sorbitol, le xylitol, le lactose.

Avantageusement, on utilisera le lactose à titre de diluant.

Les quantités respectives des différents constituants de la composition conforme à la présente invention pourront être facilement déterminées par un homme de métier, et dépendent bien entendu de la forme pharmaceutique finale souhaitée.

D'une façon générale, une composition conforme à la présente invention, destinée à la préparation d'une poudre effervescente ou non, peut contenir, exprimé en parties en poids pour 10 parties de métoclopramide ou de l'un de ses sels pharmaceutiquement acceptables :
- une quantité de sel ou complexe soluble dans l'eau d'acide acétylsalicylique équivalente à une quantité de 100 à 1000 parties en poids d'acide acétylsalicylique;
- de 2 à 15 parties en poids de polymère hydrophile; et éventuellement :
- de 50 à 400 parties en poids de déshydratant interne;
- de 50 à 600 parties en poids de système effervescent;
- de 500 à 2500 parties en poids de diluant.

Une composition préférentielle comprend, exprimée en parties en poids pour 10 parties de métoclopramide ou de l'un de ses sels pharmaceutiquement acceptables :
- une quantité de sel ou complexe soluble dans l'eau d'acide acétylsalicylique équivalente à une quantité de 800 à 1000 parties en poids d'acide acétylsalicylique;
- de 2 à 10 parties en poids de polyvidone; et éventuellement :
- de 70 à 180 parties en poids de déshydratant interne;
- de 200 à 500 parties en poids de système effervescent;
- de 750 à 1750 parties en poids de diluant.

Une composition particulièrement préférée, comprend exprimé en poids pour 10 parties de métoclopramide ou de l'un de ses sels pharmaceutiquement acceptables :
- une quantité de sel ou complexe soluble dans l'eau d'acide acétylsalicylique équivalente à une quantité de 900 parties en poids d'acide acétylsalicylique;
- 5 parties en poids de polyvidone; et éventuellement :
- 180 parties en poids de déshydratant interne;
- de 200 à 400 parties enpoids de système effervescent;
- 1500 parties en poids de diluant.

Selon un deuxième aspect, la présente invention a pour objet une préparation pharmaceutique, sous la forme d'une poudre, effervescente ou non, caractérisée en ce qu'elle contient une composition telle que définie précédemment éventuellement associée à au moins un additif usuel choisi parmi les édulcorants, les aromatisants, les colorants et les lubrifiants.

Le choix de ces additifs et de leurs quantités respectives, pourra être facilement déterminé par un homme de métier.

Un édulcorant peut être un sucre naturel, comme par exemple le sucrose ou le sorbitol, ou bien encore un produit de synthèse comme par exemple la saccharine ou l'aspartame.

Un édulcorant actuellement préféré est l'aspartame.

Un aromatisant actuellement préféré est l'arome artificiel de vanille.

Bien entendu, tout autre aromatisant pharmaceutiquement acceptable connu peut être utilisé dans le cadre de l'invention.

Un lubrifiant actuellement préféré est le benzoate de potassium.

Bien entendu, tout autre lubrifiant pharmaceutiquement acceptable connu peut être utilisé dans le cadre de l'invention.

Selon une caractéristique particulière, une préparation pharmaceutique conforme à la présente invention se présentera sous la forme d'une poudre contenant une quantité de composition telle que définie précédemment correspondant à 5 mg, 10 mg ou 30 mg de métoclopramide par unité posologique.

Selon un troisième aspect, la présente invention a pour objet un procédé de fabrication d'une préparation pharmaceutique sous forme de poudre, effervescente ou non, caractérisé en ce qu'il comprend :
a) le traitement du métoclopramide, ou de l'un de ses sels pharmaceutiquement acceptables, par une quantité efficace d'au moins un polymère hydrophile, de préférence par mélange de poudres et granulation;
b) le prémélange du sel ou complexe soluble dans l'eau de l'acide acétylsalicylique et d'au moins une parties des autres constituants de la forme pharmaceutique, de préférence sous forme de poudres;
c) la granulation éventuelle du prémélange issu de l'étape b) et des éventuels constituants de la forme pharmaceutique non encore incorporés;
d) le mélange des produits issus des étapes a) et b) ou c) et des éventuels constituants de la forme pharmaceutique non encore incorporés ; et éventuellement
e) la répartition en sachets de la poudre obtenue à l'issue de l'étape d).

D'une façon avantageuse, le traitement du métoclopramide ou de l'un de ses sels pharmaceutiquement acceptables, par le polymère hydrophile sera réalisé en granulant un mélange pulvérulent de ces deux constituants avec un solvant approprié.

Un tel solvant peut être l'eau, l'alcool, le dichlorométhane, l'isopropanol ou un mélange de deux ou plusieurs de ces composés.

Une variante de réalisation peut consister en une pulvérisation sur le métoclopramide ou l'un de ses sels pharmaceutiquement acceptables sous forme pulvérulente, d'une solution de polymère hydrophile dans le solvant approprié.

La granulation décrite à l'étape c) précitée est de préférence réalisée par compactage à sec, mais peut être également réalisée par une technique de granulation humide dans un solvant approprié telle que celle classiquement utilisée dans l'industrie pharmaceutique utilisant notamment un mélangeur planétaire, un mélangeur-granulateur sous vide, un lit fluidisé, un mélangeur à sec ou une turbine.

Il est à noter qu'au niveau de cette étape, le système effervescent peut être incorporé directement sous forme de granulés.

Il est également à noter que certains des constituants de la forme pharmaceutique, comme par exemple l'édulcorant ou l'aromatisant, peuvent être incorporés au cours de l'étape b) précitée, dans le cas d'un compactage à sec, mais peuvent être également mélangés directement au niveau de l'étape d) précitée.

La présente invention sera illustrée par les exemples non limitatifs suivants, dans lesquels les quantités indiquées sont exprimées en parties en poids pour 10 parties en poids de métoclopramide ou de l'un de ses sels pharmaceutiquement acceptables.

### EXEMPLE 1

### ETAPE A : TRAITEMENT DU METOCLOPRAMIDE

Un mélange pulvérulent de chlorhydrate monohydraté de métoclopramide et de polyvidone (5 parties en poids) est granulé avec 7 % d'eau purifiée (poids/poids).

### ETAPE B : PRE-MELANGE DU SEL OU COMPLEXE DE L'ACIDE ACETYLSALICYLIQUE ET DES AUTRES CONSTITUANTS

Un pré-mélange est réalisé avec les constituants suivants :
- carbasalate calcique (quantité correspondant à 900 parties en poids d'acide acétylsalicylique);
- acide citrique anhydre (168 parties en poids);
- bicarbonate de sodium (232 parties en poids);
- lactose (1500 parties en poids);
- citrate de magnésium (180 parties en poids);
- benzoate de potassium (250 parties en poids).

### ETAPE C : COMPACTAGE A SEC DU MELANGE ISSU DE L'ETAPE B.

### ETAPE D : MELANGE FINAL

Les produits issus des étapes A et C, ainsi que les composés suivants (aspartame, arôme artificiel vanille), qui se présentent sous forme de poudre, sont mélangés.

### ETAPE E : CONDITIONNEMENT EN SACHETS

Le mélange de poudres obtenu à l'issue de l'étape C peut être directement conditionné en sachets.

### EXEMPLE 2

En suivant le mode opératoire décrit ci-dessus, on a préparé des poudres effervescentes, ou non, ayant la composition suivante :

| **PRODUIT** | **Exemple 2** | **Exemple 3** | **Exemple 4** | **Exemple 5** | **Exemple 6** |
|---|---|---|---|---|---|
| Carbasalate calcique (exprimé en partie en poids d'acide acétylsalicylique) | 900 | 900 | 900 | 900 | 900 |
| Métoclopramide HCl H2O (exprimé en partie en poids de métoclopramide) | 10 | 10 | 10 | 10 | 10 |
| Polyvidone | 8 | 3 | 5 | 5 | 5 |
| Acide citrique | - | 126 | - | 294 | 168 |
| Lactose | 1000 | 1500 | 2500 | 1750 | 900 |
| Bicarbonate de sodium | - | 174 | - | 406 | 232 |
| Citrate de magnésium | - | 150 | 100 | 250 | 180 |
| Benzoate de potassium | 100 | 200 | 150 | 250 | 200 |
| Aspartame | 5 | 15 | - | 15 | 15 |
| Arôme | 25 | 30 | 25 | 30 | 30 |
| Saccharinate de sodium | 10 | - | 10 | - | - |
| Mannitol | 1310 | - | - | - | - |
| Acide tartrique | 160 | - | - | - | - |
| Carbonate de sodium | 120 | - | - | - | - |

## Revendications

1. Composition pharmaceutique destinée à la préparation d'une poudre, caractérisée en ce qu'elle comprend à titre d'ingrédient actif, une quantité efficace d'un sel ou complexe soluble dans l'eau de l'acide acétylsalicylique en association avec du métoclopramide ou l'un de ses sels pharmaceutiquement acceptables ; et au moins un polymère hydrophile pharmaceutiquement acceptable, en une quantité suffisante pour stabiliser le métoclopramide.

2. Composition selon la revendication 1, caractérisée en ce que le sel ou complexe soluble dans l'eau de l'acide acétylsalicylique précité est le carbasalate de calcium.

3. Composition selon la revendication 1 ou 2, caractérisée en ce que le polymère hydrophile précité est la polyvidone.

4. Composition selon l'une quelconque des revendications 1 à 3, caractérisée en ce qu'elle comprend en outre un système effervescent pharmaceutiquement acceptable comprenant au moins un acide organique et au moins une substance susceptible de réagir avec cet acide organique pour produire un dégagement gazeux de dioxyde de carbone.

5. Composition selon l'une quelconque des revendications 1 à 4, caractérisée en ce qu'elle comprend en outre une quantité efficace d'au moins un déshydratant interne.

6. Composition selon la revendication 5, caractérisée en ce que le déshydratant interne précité est le citrate de magnésium anhydre.

7. Composition selon l'une quelconque des revendications 1 à 6, caractérisée en ce qu'elle comprend en outre un diluant pharmaceutiquement acceptable.

8. Composition selon la revendication 7, caractérisée en ce que le diluant précité est le lactose.

9. Composition selon l'une quelconque des revendications 1 à 8, caractérisée en ce qu'elle comprend, exprimé en parties en poids, pour 10 parties de métoclopramide ou de l'un de ses sels pharmaceutiquement acceptables :
- une quantité de sel ou complexe soluble dans l'eau d'acide acétylsalicylique équivalente à une quantité de 100 à 1000 parties en poids d'acide acétylsalicylique;
- de 2 à 15 parties en poids de polymère hydrophile; et éventuellement :
- de 50 à 400 parties en poids de déshydratant interne;
- de 50 à 600 parties en poids de système effervescent;
- de 500 à 2500 parties en poids de diluant.

10. Composition selon la revendication 9, caractérisée en ce qu'elle comprend, exprimé en parties en poids, pour 10 parties de métoclopramide ou de l'un de ses sels pharmaceutiquement acceptables :
- une quantité de sel ou complexe soluble dans l'eau d'acide acétylsalicylique équivalente à une quantité de 800 à 1000 parties en poids d'acide acétylsalicylique;
- de 2 à 10 parties en poids de polyvidone; et éventuellement :
- de 70 à 180 parties en poids de déshydratant interne;
- de 200 à 500 parties en poids de système effervescent;
- de 750 à 1750 parties en poids de diluant.

11. Composition selon la revendication 10, caractérisée en ce qu'elle comprend, exprimé en parties en poids, pour 10 parties de métoclopramide ou de l'un de ses sels pharmaceutiquement acceptables :
- une quantité de sel ou complexe soluble dans l'eau d'acide acétylsalicylique équivalente à une quantité de 900 parties en poids d'acide acétylsalicylique;
- 5 parties en poids de polyvidone; et éventuellement :
- 180 parties en poids de déshydratant interne;
- de 200 à 400 parties en poids de système effervescent;
- 1500 parties en poids de diluant.

12. Préparation pharmaceutique, sous la forme d'une poudre, caractérisée en ce qu'elle contient une composition telle que définie à l'une quelconque des revendications 1 à 11, éventuellement associée à au moins un additif usuel choisi parmi les édulcorants, les aromatisants, les colorants et lubrifiants.

13. Préparation pharmaceutique selon la revendication 12, caractérisée en ce qu'elle contient une quantité de composition telle que définie à l'une quelconque des revendications 1 à 12 correspondant à 5 mg, 10 mg ou 30 mg de métoclopramide par unité posologique.

14. Procédé de fabrication d'une préparation pharmaceutique sous forme de poudre, telle que définie à la revendiction 12 ou 13, caractérisé en ce qu'il comprend :
a) le traitement du métoclopramide, ou de l'un de ses sels pharmaceutiquement acceptables, par une quantité efficace d'au moins un polymère hydrophile, de préférence par mélange de poudres et granulation;
b) le prémélange du sel ou complexe soluble dans l'eau de l'acide acétylsalicylique et d'au moins une parties des autres constituants de la forme pharmaceutique, de préférence sous forme de poudres;
c) la granulation éventuelle du prémélange issu de l'étape b) et des éventuels constituants de la forme pharmaceutique non encore incorporés;
d) le mélange des produits issus des étapes a) et b) ou c) et des éventuels constituants de la forme pharmaceutique non encore incorporés ; et éventuellement
e) la répartition en sachets de la poudre obtenue à l'issue de l'étape d).

## Claims

1. A pharmaceutical composition for the preparation of a powder, said composition comprising, as the active ingredient, an effective amount of a water-soluble salt or complex of acetylsalicylic acid in association with metoclopramid or one of its pharmaceutically acceptable salts, and at least one pharmaceutically acceptable hydrophilic polymer in a sufficient amount to stabilize the metoclopramid.

2. A composition according to claim 1 wherein the above-mentioned water-soluble salt or complex of acetylsalicylic acid is carbasalate calcium.

3. A composition according to claim 1 or 2 wherein the above-mentioned hydrophilic polymer is polyvidone.

4. A composition according to any one of claims 1 to 3 which also comprises a pharmaceutically acceptable effervescent system containing at least one organic acid and at least one substance capable of reacting with this organic acid to release carbon dioxide gas.

5. A composition according to any one of claims 1 to 4 which also comprises an effective amount of at least one internal dehydrating agent.

6. A composition according to claim 5 wherein the above-mentioned internal dehydrating agent is anhydrous magnesium citrate.

7. A composition according to any one of claims 1 to 6 which also comprises a pharmaceutically acceptable diluent.

8. A composition according to claim 7 wherein the above-mentioned diluent is lactose.

9. A composition according to any one of claims 1 to 8 which comprises the following, expressed in parts by weight per 10 parts of metoclopramid or one of its pharmaceutically acceptable salts:
- water-soluble salt or complex of acetylsalicylic acid in an amount equivalent to 100 to 1000 parts by weight of acetylsalicylic acid;
- from 2 to 15 parts by weight of hydrophilic polymer; and if appropriate:
- from 50 to 400 parts by weight of internal dehydrating agent;
- from 50 to 600 parts by weight of effervescent system;
- from 500 to 2500 parts by weight of diluent.

10. A composition according to claim 9 which comprises the following, expressed in parts by weight per 10 parts of metoclopramid or one of its pharmaceutically acceptable salts:
- water-soluble salt or complex of acetylsalicylic acid in an amount equivalent to 800 to 1000 parts by weight of acetylsalicylic acid;
- from 2 to 10 parts by weight of polyvidone; and if appropriate:
- from 70 to 180 parts by weight of internal dehydrating agent;
- from 200 to 500 parts by weight of effervescent system;
- from 750 to 1750 parts by weight of diluent.

11. A composition according to claim 10 which comprises the following, expressed in parts by weight per 10 parts of metoclopramid or one of its pharmaceutically acceptable salts:
- water-soluble salt or complex of acetylsalicylic acid in an amount equivalent to 900 parts by weight of acetylsalicylic acid;
- 5 parts by weight of polyvidone;
and if appropriate:
- 180 parts by weight of internal dehydrating agent;
- from 200 to 400 parts by weight of effervescent system;
- 1500 parts by weight of diluent.

12. A pharmaceutical preparation in powder form, which contains a composition as defined in any one of claims 1 to 11, optionally associated with at least one customary additive selected from sweeteners, flavorings, colors and lubricants.

13. A pharmaceutical preparation according to claim 12 which contains an amount of composition as defined in any one of claims 1 to 12 corresponding to 5 mg, 10 mg or 30 mg of metoclopramid per dosage unit.

14. A process for the manufacture of a pharmaceutical preparation in powder form, as defined in claim 12 or 13, said process comprising:
a) treating the metoclopramid or one of its pharmaceutically acceptable salts with an effective amount of at least one hydrophilic polymer, preferably by the mixing of powders and granulation;
b) premixing the water-soluble salt or complex of acetylsalicylic acid and at least part of the other constituents of the pharmaceutical form, preferably in the form of powders;
c) if appropriate, granulating the premix resulting from step b) and any constituents of the pharmaceutical form which have not yet been incorporated;
d) mixing the products resulting from steps a) and b) or c) and any constituents of the pharmaceutical form which have not yet been incorporated; and if appropriate
e) distributing the powder obtained at the end of step
d) into sachets.

## Patentansprüche

1. Pharmazeutische Zusammensetzung zur Herstellung eines Pulvers, **dadurch gekennzeichnet, daß** sie als Wirkstoff eine wirksame Menge eines wasserlöslichen Salzes oder Komplexes von Acetylsalicylsäure in Kombination mit Metoclopramid oder einem seiner pharmazeutisch verträglichen Salze und mindestens ein pharmazeutisch verträgliches hydrophiles Polymer in einer Menge enthält, die ausreichend ist, um das Metoclopramid zu stabilisieren.

2. Zusammensetzung nach Anspruch 1, **dadurch gekennzeichnet, daß** das wasserlösliche Salz oder der wasserlösliche Komplex der Acetylsalicylsäure Carbasalat-Calcium ist.

3. Zusammensetzung nach Anspruch 1 oder 2, **dadurch gekennzeichnet, daß** das hydrophile Polymer Polyvidon ist.

4. Zusammensetzung nach einem der Ansprüche 1 bis 3, **dadurch gekennzeichnet, daß** sie außerdem ein pharmazeutisch verträgliches Brause-bildendes System umfaßt, das mindestens eine organische Säure und mindestens eine Substanz enthalt, die in der Lage ist, mit der organischen Säure zu reagieren, wobei gasförmiges Kohlendioxid freigesetzt wird.

5. Zusammensetzung nach einem der Ansprüche 1 bis 4, **dadurch gekennzeichnet, daß** sie darüber hinaus eine wirksame Menge mindestens eines inneren Entwässerungsmittels enthält.

6. Zusammensetzung nach Anspruch 5, **dadurch gekennzeichnet, daß** das innere Entwässerungsmittel wasserfreies Magnesiumcitrat ist.

7. Zusammensetzung nach einem der Ansprüche 1 bis 6, **dadurch gekennzeichnet, daß** sie zusätzlich ein pharmazeutisch verträgliches Streckungsmittel enthält.

8. Zusammensetzung nach Anspruch 7, **dadurch gekennzeichnet, daß** das Streckungsmittel Lactose ist.

9. Zusammensetzung nach einem der Ansprüche 1 bis 8, **dadurch gekennzeichnet, daß** sie in Gewichtsteilen ausgedrückt auf 10 Teile Metoclopramid oder eines seiner pharmazeutisch verträglichen Salze
- eine äquivalente Menge des wasserlöslichen Salzes oder Komplexes der Acetylsalicylsäure mit einer Menge von 100 bis 1000 Gew.-Teilen Acetylsalicylsäure,
- 2 bis 15 Gew.-Teile hydrophiles Polymer
und gegebenenfalls
- 50 bis 400 Gew.-Teile inneres Entwässerungsmittel,
- 50 bis 600 Gew.-Teile an Brause-bildendem System und
- 500 bis 2500 Gew.-Teile Streckungsmittel
enthält.

10. Zusammensetzung nach Anspruch 9, **dadurch gekennzeichnet, daß** sie in Gewichtsteilen ausgedrückt auf 10 Teile Metoclopramid oder eines seiner pharmazeutisch verträglichen Salze
- eine äquivalente Menge des wasserlöslichen Salzes oder Komplexes der Acetylsalicylsäure mit einer Menge von 800 bis 1000 Gew.-Teilen Acetylsalicylsäure,
- 2 bis 10 Gew.-Teile Polyvidon,
und gegebenenfalls
- 70 bis 180 Gew.-Teile inneres Entwässerungsmittel,
- 200 bis 500 Gew.-Teile an Brause-bildendem System und
- 750 bis 1750 Gew.-Teile Streckungsmittel
enthält.

11. Zusammensetzung nach Anspruch 10, **dadurch gekennzeichnet, daß** sie in Gewichtsteilen ausgedrückt auf 10 Teile Metoclopramid oder eines seiner pharmazeutisch verträglichen Salze
- eine äquivalente Menge des wasserlöslichen Salzes oder Komplexes der Acetylsalicylsäure mit einer Menge von 900 Gew.-Teilen Acetylsalicylsäure,
- 5 Gew.-Teile Polyvidon,
und gegebenenfalls
- 180 Gew.-Teile inneres Entwässerungsmittel,
- 200 bis 400 Gew.-Teile an Brause-bildendem System und
- 1500 Gew.-Teile Streckungsmittel
enthält.

12. Arzneimittelzubereitung in Form eines Pulvers, **dadurch gekennzeichnet, daß** sie eine Zusammensetzung gemäß einem der Ansprüche 1 bis 11, gegebenenfalls mit mindestens einem üblichen Zusatzstoff kombiniert, enthält, der aus Süßstoffen, Aromatisierungs-, Farb- und Gleitmitteln ausgewählt ist.

13. Arzneimittelzubereitung nach Anspruch 12, **dadurch gekennzeichnet, daß** sie eine Menge der Zusammensetzung gemäß einem der Ansprüche 1 bis 12 enthält, die 5 mg, 10 mg oder 30 mg Metoclopramid pro Dosierungseinheit entspricht.

14. Verfahren zur Herstellung einer Arzneimittelzubereitung in Form eines Pulvers gemäß Anspruch 12 oder 13, **dadurch gekennzeichnet, daß** es umfaßt
a) die Behandlung des Metoclopramids oder eines seiner pharmazeutisch verträglichen Salze mit einer wirksamen Menge mindestens eines hydrophilen Polymers, vorzugsweise durch Vermischen der Pulver und Granulieren,
b) Vormischen des wasserlöslichen Salzes oder Komplexes der Acetylsalicylsäure mit mindestens einem Teil der anderen Bestandteile der Arzneiform, vorzugsweise als Pulver,
c) gegebenenfalls Granulieren des Vorgemischs aus Stufe b) und der möglichen noch nicht eingebauten Bestandteile der Arzneiform,
d) Vermischen der Produkte aus den Stufen a) und b) oder c) und möglicher noch nicht eingebauter Bestandteile der Arzneiform und gegebenenfalls
e) Portionierung des in Stufe d) hergestellten Pulvers in Beutel.
